# EUROPEAN PATENT APPLICATION

(11) **EP 4 527 825 A1**
(43) Date of publication of application: **26.03.2025**
(21) Application number: 23807394.4
(22) Date of filing: 25.04.2023
(51) Int. Cl.: C07C 1/12, C07C 9/04, C07B 61/00, B01J 8/04

(54) **REACTION DEVICE**

(30) Priority: 17.05.2022 JP 2022080745
(71) Applicant: Niterra Co., Ltd., Nagoya-shi, Aichi 461-0005 (JP)
(72) Inventor: NAKAMURA Yosuke, Nagoya-shi, Aichi 461-0005 (JP); YABUHANA Masaki, Nagoya-shi, Aichi 461-0005 (JP); FUNAHASHI Yoshihiro, Nagoya-shi, Aichi 461-0005 (JP)
(74) Representative: Grünecker Patent- und Rechtsanwälte PartG mbB
(86) International application number: PCT/JP2023/016259
(87) International publication number: WO 2023/223783

(57) **Abstract**

To provide a reaction device in which deterioration of a catalyst is suppressed and which can employ a short reaction vessel. A reaction device (10) in which an exothermic chemical reaction occurs, has a reaction vessel (20) through which a source gas passes from an inlet to an outlet, and catalysts (24, 26) accommodated in the reaction vessel. The reaction device includes a first section (21), a second section (22), and a third section (23) disposed in that sequence along the direction of flow of the source gas. Among the first, second, and third sections, the degree of lowering the activation energy of the chemical reaction is the smallest in the second section. **In** the reaction device, the temperature rises in the first section, lowers in the second section, and rises in the third section.

## Description

### TECHNICAL FIELD

The present invention relates to a reaction device in which an exothermic chemical reaction is conducted.

### BACKGROUND ART

In a reaction device which has a reaction vessel including a catalyst and through which a source gas is caused to pass, resulting in chemical reaction accompanied by heat generation, the temperature of the reaction field rises by heat of reaction, whereby deterioration of the catalyst (e.g., sintering) readily occurs. Patent Literature 1 discloses a related technique for suppressing deterioration of such a catalyst. In the technique, source gases are caused to pass through a series of reaction vessels that are juxtaposed at specific intervals, and the gases are combined, to thereby reduce variation in temperature distribution in a cross-sectional direction of each reaction vessel. As a result, overheating of the catalyst is prevented.

### CITATION LIST

### PATENT LITERATURE

Patent Literature 1: JP2018-114432A

### SUMMARY OF INVENTION

### TECHNICAL PROBLEM

In the related technique, reaction vessels are juxtaposed at specific intervals, and the total volume of the reaction vessels is smaller than the volume of the space in which the reaction vessels are disposed. When the total volume of reaction vessels commensurate with the volume of the space in which the reaction vessels are disposed is secured, the total path length of the reaction vessels problematically increases.

The present invention has been accomplished to solve the above problem. Thus, an object of the present invention is to provide a reaction device which can achieve reduction of deterioration of a catalyst and shorten the path length of a reaction vessel.

### SOLUTION TO PROBLEM

In a first aspect of the present invention so as to attain the above object, there is provided a reaction device in which an exothermic chemical reaction occurs, the reaction device having a reaction vessel through which a source gas passes from an inlet to an outlet, and a catalyst accommodated in the reaction vessel. The reactor includes a first section, a second section, and a third section disposed in that sequence along the direction of flow of the source gas. Among the first, second, and third sections, the degree of lowering the activation energy of the chemical reaction is the smallest in the second section.

In a second aspect of the present invention, there is provided a reaction device in which an exothermic chemical reaction occurs, the reaction device having a reaction vessel through which a source gas passes from an inlet to an outlet, and a catalyst accommodated in the reaction vessel. The reactor includes a first section, a second section, and a third section disposed in that sequence along the direction of flow of the source gas. The temperature rises in the first section, lowers in the second section, and rises in the third section.

A third aspect is directed to a specific embodiment of the first and second aspects, in which the second section accommodates an inert body having no catalytic activity or a low-activity catalyst having a catalytic activity lower than that of a catalyst accommodated in the first section or the third section.

A fourth aspect is directed to a specific embodiment of any of the first to third aspects, in which the first section accommodates a catalyst having a catalytic activity lower than that of a catalyst accommodated in the third section.

A fifth aspect is directed to a specific embodiment of any of the first to fourth aspects, in which the first section has a thickness in the gas flow direction smaller than the thickness of the third section in the gas flow direction.

A sixth aspect is directed to a specific embodiment of any of the first to fifth aspects, in which the chemical reaction is a reaction of synthesizing methane from a source gas including hydrogen and carbon dioxide.

### ADVANTAGEOUS EFFECTS OF INVENTION

The reaction device has a reaction vessel and a catalyst accommodated in the reaction vessel, and includes a first section, a second section, and a third section disposed in that sequence along the direction of flow of the source gas. Among the first, second, and third sections, the degree of lowering the activation energy of the chemical reaction is the smallest in the second section. Thus, heat generation in the second section is smaller than that in the first section or the third section. Since the temperature rose in the first section lowers in the second section, over-heating of the catalyst can be prevented, even the path length of a reaction vessel is short. As a result, deterioration of the catalyst can be suppressed.

The reaction device has a reaction vessel and a catalyst accommodated in the reaction vessel, and includes a first section, a second section, and a third section disposed in that sequence along the direction of flow of the source gas. The temperature rises in the first section, lowers in the second section, and rises in the third section. Over-heating of the catalyst can be prevented, even the path length of a reaction vessel is short. As a result, deterioration of the catalyst can be suppressed.

### BRIEF DESCRIPTION OF DRAWINGS

[FIG. 1] A block diagram of a reaction device of a first embodiment.
[FIG. 2] A cross-section of a reaction vessel.
[FIG. 3] A graph showing a temperature in each section of the reaction device.
[FIG. 4] A cross-section of a reaction device of a second embodiment.
[FIG. 5] A cross-section of a reaction device of a third embodiment.
[FIG. 6] A cross-section of a reaction device of a fourth embodiment.

### DESCRIPTION OF EMBODIMENTS

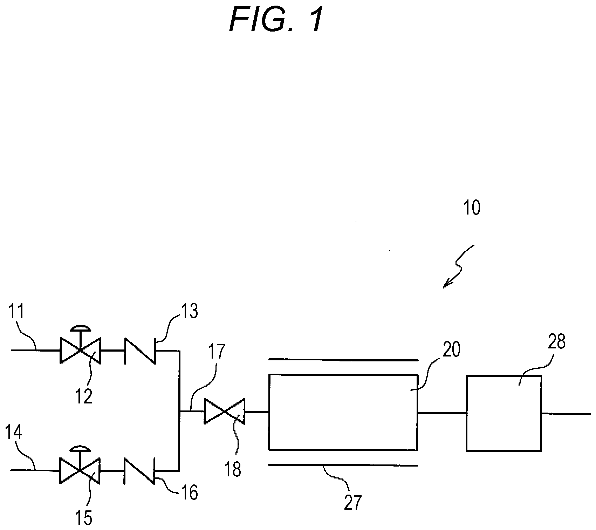

With reference to the attached drawings, a preferred embodiments of the present invention will be described. FIG. 1 is a block diagram of a reaction device 10 of a first embodiment. The reaction device 10 has a reaction vessel 20 in which a source gas passes. The reaction vessel 20 is connected to a confluence pipe 17, to which two pipes 11, 14 are connected. The pipe 11 is a pipe through which a first source gas is supplied, and a control valve 12 and a check valve 13 are disposed in that sequence from the upstream side to the downstream side. The pipe 14 is a pipe through which a second source gas is supplied, and a control valve 15 and a check valve 16 are disposed in that sequence from the upstream side to the downstream side. The confluence pipe 17 is provided with a gate valve 18. By means of the control valves 12, 15, the first and second source gases are mixed at an optimum mixing ratio. The thus obtained gas mixture (i.e., a source gas) is supplied to the reaction vessel 20 via the gate valve 18.

The present embodiment corresponds to a case in which the first source gas is hydrogen, and the second source gas is carbon dioxide. The embodiment will be described. In the reaction vessel 20, pressure is appropriately controlled, and the container is heated by means of a heater 27, whereby production of methane (i.e., methanation) represented by the chemical reaction formula: CO₂ + 4H₂ → CH₄ + 2H₂O is conducted. The product is cooled by means of a condenser 28 connected to a downstream side of the reaction vessel 20 to ice temperature. As a result, the product is separated into water and a methane-containing gas. The methane-containing gas may contain hydrogen and carbon dioxide serving as source gases.

Methanation is an example of the chemical reaction occurring in the reaction device 10, and the chemical reaction is not limited thereto. By appropriately modifying the source gas, reaction conditions, and the catalyst (mentioned hereinbelow), for example, the following chemical reactions can be caused to be conducted in the reaction device 10.

Production of syngas via partial oxidation of methane: 2CH₄ + O₂ → 2CO + 4H₂
Methanol synthesis: CO + 2H₂ → CH₃OH
Methanol synthesis: CO₂ + 3H₂ → CH₃OH + H₂O
Fischer-Tropsch synthesis: CO + 2H₂ → -(CH₂)- + H₂O (-(CH₂)- represents a linear-chain hydrocarbon)
Dimethyl ether synthesis: 2CO + 4H₂ → CH₃OCH₃ + H₂O

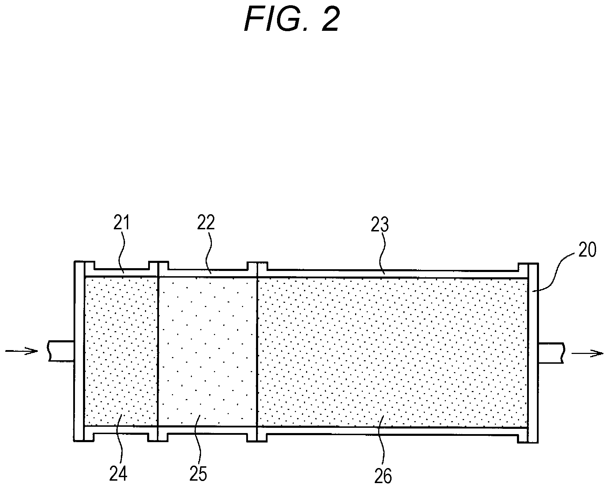

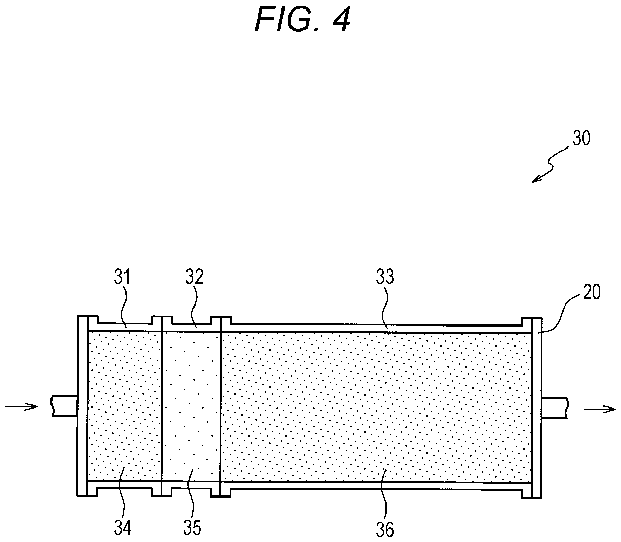

FIG. 2 is a cross-section of the reaction device 10. The arrows shown in FIG. 2 denote the flow direction of the source gas reaction in the container 20 (the same applies to FIGs. 4 to 6). The reaction vessel 20 accommodates catalysts 24, 26, and a low-activity catalyst 25. The reaction device 10 includes a first section 21, a second section 22, and a third section 23 disposed in that sequence along the direction of flow of the source gas (upstream side to downstream side). The first section 21 and the third section 23 accommodate the catalysts 24, 26. The second section 22 accommodates the low-activity catalyst 25 having a catalytic activity lower than that of the catalyst 24 or 26. The catalysts 24, 26, and the low-activity catalyst 25 lower the activation energy of chemical reaction, to thereby facilitate progress of the chemical reaction. A low catalytic activity refers to a small degree of lowering the activation energy of chemical reaction.

In the present embodiment, the first section 21, the second section 22, and the third section 23 are joined to one another by fastening flanges of tubes having the same inner diameter with bolts or the like, the tubes accommodating the catalyst 24, the low-activity catalyst 25, and the catalyst 26, respectively. However, the fastening manner is not limited thereto. Needless to say, alternatively, the catalyst 24, the low-activity catalyst 25, and the catalyst 26 may be sequentially charged to a single tube, to thereby provide the first section 21, the second section 22, and the third section 23.

No particular limitation is imposed on the catalysts 24, 26, and the low-activity catalyst 25 used in the embodiment, so long as the catalysts are adapted to various chemical reaction. Examples of the catalysts 24, 26, and the low-activity catalyst 25 include powder, pellets, and a porous body of a particle-on-carrier. Examples of the carrier include powder, pellets, and a porous body of an oxide including one or more species of alumina, silica, magnesia, titania, zirconia, niobia, silica-alumina, zeolite, and calcium phosphate. The porous body has gas permeability which allows the source gas to pass through. In the case of powder or pellets, the source gas passes through voids therein.

Examples of the material of the particles supported on the carrier include metals including one or more elements of Fe, Co, Ni, Cu, Ru, Rh, Pd, Ag, Ir, Pt, and Au. Regarding the catalyst, if the same material and particle size of the carrier and particles are employed, catalytic activity increases in proportion to the surface area of the particles supported on the carrier. Thus, by reducing the surface area of the particles supported on the carrier with respect to that of the catalysts 24, 26, the low-activity catalyst 25 can be provided.

Also, when inert particles having no catalytic activity are added to the catalyst 24 or 26 so as to reduce the relative amount of catalyst 24 or 26 in a specific amount, the low-activity catalyst 25 is provided. Examples of the inert particle species include powder and pellets of an oxide including one or more species of alumina, silica, magnesia, titania, zirconia, niobia, silica-alumina, zeolite, and calcium phosphate.

When a source gas including hydrogen and carbon dioxide is caused to pass through the reaction vessel 20, the source gas passes through the first section 21, the second section 22, and the third section 23 in that sequence, whereby a chemical reaction forming methane and water proceeds.

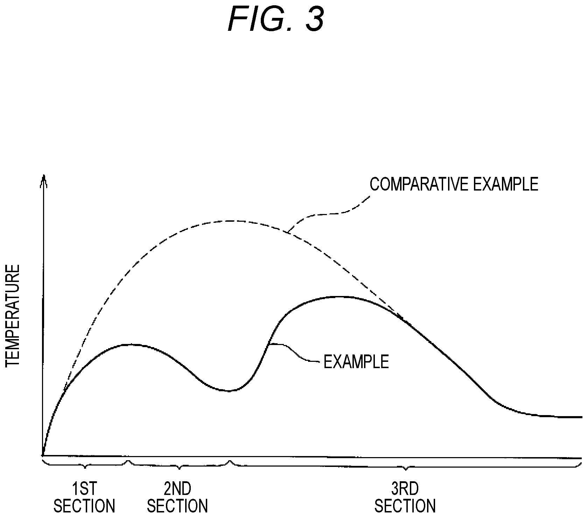

FIG. 3 is a graph showing a change in temperature over the sections of the reaction device 10. An Example in relation to FIG. 3 corresponds to temperatures recorded at the first section 21, the second section 22, and the third section 23. The temperatures are sensed by a plurality of thermocouples disposed in the source gas flow direction along the center axis penetrating the cross-sections of reaction field (catalyst present area) of the first to third sections. A Comparative Example corresponds to temperatures recorded in a similar manner, but the reaction device 10 is not segmented into the first section 21, the second section 22, and the third section 23, and the catalyst 24 of the first section 21 is disposed in the whole area of the reaction vessel 20.

As shown in FIG. 3, in the Example, the temperature rises in the first section 21, lowers in the second section 22, and rises in the third section 23. In the second section 22, the catalytic activity of the low-activity catalyst 25 is lower than that of the catalyst 24, and the degree of lowering activation energy is smaller than that in the first section 21. As a result, the reaction rate in the second section 22 is less than that in the first section 21, leading to a drop in temperature. In the third section 23, the catalytic activity of the activity catalyst 26 is higher than that of the low-activity catalyst 25, and the degree of lowering activation energy is greater than that in the second section 22. Thus, the rate of reaction becomes greater than that in the second section 22, leading to a rise in temperature by heat of reaction. However, since methane and steam (i.e., reaction products) are present in the third section 23, heat of reaction in the third section 23 does not considerably increase. Also, since the second section 22 intervenes between the third section 23 and the first section 21, the heat in the third section 23 is not easily transferred to the first section 21. As a result, the temperature in the first section 21 does not considerably rise.

In contrast, in the Comparative Example, the maximum arrival temperature of the reaction field by a chemical reaction forming methane and water from a source gas (exothermic reaction) is higher than that attained in the Example. In the Example, the temperatures of the catalysts 24, 26 and the low-activity catalyst 25, which are elevated as the progress of chemical reaction, can be lowered, as compared with the Comparative Example, leading to reduction in deterioration of the catalysts. In addition, since juxtaposing reaction vessels at specific intervals as employed in the related art is not needed, the total pass length of the reaction vessel 20 can be reduced, while the volume of the reaction vessel 20 is secured.

The embodiment will be described with reference to FIG.2 again. The catalytic activity of the catalyst 24 in the first section 21 is equal to that of the catalyst 26 in the third section 23, or lower than that of the catalyst 26 in the third section 23. The thickness of the first section 21 in the source gas flow direction is smaller than that of the third section 23 in the source gas flow direction. Thus, even when the catalytic activity of the catalyst 24 in the first section 21 is equivalent to the catalytic activity of the catalyst 26 in the third section 23, the temperature of the first section 21 attributed to heat of reaction can be lowered, as compared with the case in which the thickness of the first section 21 is greater than that of the third section 23. As a result, deterioration of the catalyst 24 can be suppressed. If catalysts having the same catalytic activity are placed in the first section 21 and the third section 23, respectively, two catalysts 24, 26 having different catalytic activity are not necessarily provided.

The thickness of the second section 22 in the source gas flow direction is smaller than that of the third section 23 in the source gas flow direction. Thus, the total path length of the reaction vessel 20 can be shortened, as compared with the case in which the thickness of the second section 22 is greater than that of the third section 23.

The thickness of the second section 22 in the source gas flow direction is greater than that of the first section 21 in the source gas flow direction. Thus, adiabatic property of low-activity catalyst 25 disposed between the first section 21 and the third section 23 can be secured.

With reference to FIG. 4, a reaction device 30 of a second embodiment will be described. In the first embodiment, the case in which the low-activity catalyst 25 is disposed in the second section 22 was described. However, in the second embodiment, the case in which an inert body 35 having no catalytic activity is disposed in a second section 32 will be described. The same members as described in the first embodiment are denoted by the same reference numerals, and overlapping description will be omitted. FIG. 4 is a cross-section of the reaction device 30 of the second embodiment.

The reaction device 30 includes a first section 31, a second section 32, and a third section 33 in the source gas flow direction in that sequence. The reaction vessel 20 accommodates catalysts 34, 36 and the inert body 35. The first section 31 and the third section 33 accommodate the catalysts 34, 36, respectively. The catalysts 34, 36 reduce activation energy of chemical reaction, to thereby facilitate progress of the chemical reaction. The second section 32 accommodates the inert body 35 having no catalytic activity. The term "having no catalytic activity" refers to that the inert body 35 is impotent to reduce activation energy of chemical reaction.

Examples of the inert body 35 include powder, pellets, and a porous body of an oxide including one or more species of alumina, silica, magnesia, titania, zirconia, niobia, silica-alumina, zeolite, and calcium phosphate. The porous body has gas permeability which allows the source gas to pass through. In the case of powder or pellets, the source gas passes through voids therein.

When a source gas formed by mixing hydrogen with carbon dioxide is caused to pass through the reaction vessel 20, the source gas passes through the first section 31, the second section 32, and the third section 33 in that sequence, whereby a chemical reaction forming methane and water proceeds. In the second section 32, the inert body 35 has no catalytic activity, and the degree of lowering activation energy is smaller than that in the first section 31. Thus, the rate of reaction becomes smaller than that in the first section 31, resulting in a drop in temperature. In the third section 33, by virtue of the presence of the catalyst 36, the degree of lowering activation energy is greater than that in the second section 32, and the rate of reaction becomes greater than that in the second section 32, leading to a rise in temperature by heat of reaction. However, since methane and steam are present in the third section 33, heat of reaction in the third section 33 does not considerably increase. Also, since the second section 32 intervenes between the third section 33 and the first section 31, the heat in the third section 33 is not easily transferred to the first section 31. As a result, the temperature in the first section 31 does not considerably rise. Thus, deterioration of the catalysts 34, 36 can be suppressed.

The catalytic activity of the catalyst 34 in the first section 31 is equal to that of the catalyst 36 in the third section 33, or lower than that of the catalyst 36 in the third section 33. The thickness of the first section 31 in the source gas flow direction is smaller than that of the third section 33 in the source gas flow direction. The thickness of the second section 32 in the source gas flow direction is smaller than that of the third section 33 in the source gas flow direction.

The thickness of the second section 32 in the source gas flow direction is smaller than that of the first section 31 in the source gas flow direction. Thus, the total path length of the reaction vessel 20 can be shortened, as compared with the case in which the thickness of the second section 32 is greater than that of the first section 31.

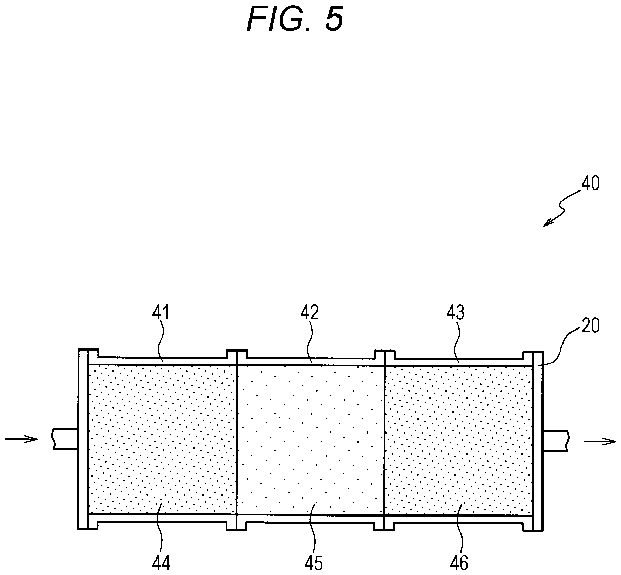

With reference to FIG. 5, a reaction device 40 of a third embodiment will be described. In the first and second embodiments, the case in which the thickness of the first section 21 or 31 is smaller than that of the third section 23 and 33 was described. However, in the third embodiment, the case in which the thickness of the first section 41 is equal to that of the third section 43 will be described. The same members as described in the first embodiment are denoted by the same reference numerals, and overlapping description will be omitted. FIG. 5 is a cross-section of the reaction device 40 of the third embodiment.

The reaction device 40 includes a first section 41, a second section 42, and a third section 43 in the source gas flow direction in that sequence. The reaction vessel 20 accommodates catalysts 44, 46 and a low-activity catalyst 45. The first section 31 and the third section 33 accommodate the catalysts 34, 36, respectively. The first section 41 and the third section 43 accommodate the catalysts 44, 46, respectively. the catalytic activity of the catalyst 44 is lower than that of the catalyst 46. The second section 42 accommodate the low-activity catalyst 45 having a catalytic activity lower than that of the catalyst 44 or 46.

When a source gas formed by mixing hydrogen with carbon dioxide is caused to pass through the reaction vessel 20, the source gas passes through the first section 41, the second section 42, and the third section 43 in that sequence, whereby a chemical reaction forming methane and water proceeds. In the second section 42, the low-activity catalyst 45 has a catalytic activity lower than that of the catalyst 44, and the degree of lowering activation energy is smaller than that in the first section 41. Thus, the rate of reaction becomes smaller than that in the first section 41, resulting in a drop in temperature. In the third section 43, by virtue of the presence of the catalyst 46, the degree of lowering activation energy is greater than that in the second section 42, and the rate of reaction becomes greater than that in the second section 42, leading to a rise in temperature by heat of reaction. However, since methane and steam are present in the third section 43, heat of reaction in the third section 43 does not considerably increase. Also, since the second section 42 intervenes between the third section 43 and the first section 41, the heat in the third section 43 is not easily transferred to the first section 41. As a result, the temperature in the first section 41 does not considerably rise. Thus, deterioration of the catalysts 44, 46 and the low-activity catalyst 46 can be suppressed.

Although the thickness of the first section 41 in the source gas flow direction is equal to that of the third section 43 in the source gas flow direction, the catalytic activity of the catalyst 44 in the first section 41 is lower than that of the catalyst 46 in the third section 43. Thus, an excessive increase in heat of reaction can be prevented in the first section 41.

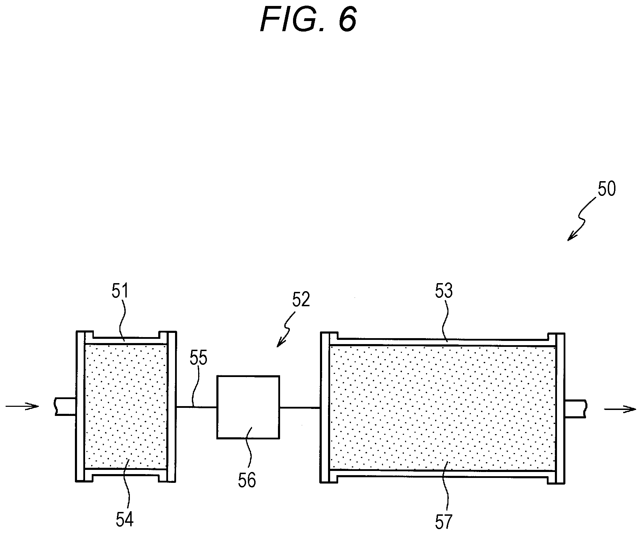

With reference to FIG. 6, a reaction device 50 of a fourth embodiment will be described. In the first to third embodiments, the cases in which the low-activity catalyst 25, 45, and the inert body 35 are accommodated in the second section 22, 32, and 42, respectively, were described. However, in the fourth embodiment, the case in which a second section 52 is equipped with a pipe 55 will be described. The same members as described in the first embodiment are denoted by the same reference numerals, and overlapping description will be omitted. FIG. 6 is a cross-section of the reaction device 50 of the fourth embodiment.

The reaction device 50 includes a first section 51, a second section 52, and a third section 53 in the source gas flow direction in that sequence. The first section 51 and the third section 53 accommodate catalyst 54, 57, respectively. The second section 52 is provided with the pipe 55 which connects the first section 51 with the third section 53, and a condenser 56 disposed around the pipe 55. The pressure in the first section 51 or the third section 53 is appropriately regulated, and the two sections are heated by means of a heater (not illustrated). No heater is disposed around the condenser 56.

When a source gas formed by mixing hydrogen with carbon dioxide is supplied to the first section 51, the source gas passes through the first section 51, the second section 52, and the third section 53 in that sequence, whereby a chemical reaction forming methane and water proceeds. In the second section 52, no catalyst is present, and the degree of lowering activation energy is smaller than that in the first section 51. Thus, the rate of reaction becomes smaller than that in the first section 51, resulting in a drop in temperature. **In** the third section 53, by virtue of the presence of the catalyst 57, the degree of lowering activation energy is greater than that in the second section 52, and the rate of reaction becomes greater than that in the second section 52, leading to a rise in temperature by heat of reaction. However, since methane are present in the third section 53, heat of reaction in the third section 53 does not considerably increase. Also, since the second section 52 intervenes between the third section 53 and the first section 51, the heat in the third section 53 is not easily transferred to the first section 51. As a result, the temperature in the first section 51 does not considerably rise. Thus, deterioration of the catalysts 54, 57 can be suppressed.

Particularly, the condenser 56 is disposed around the pipe 55, but no heater is disposed around the condenser 56. Thus, the second section 52 can be more effectively cooled, as compared with the second sections 22, 32, and 42 of the first to third embodiments.

The catalytic activity of the catalyst 54 in the first section 51 is equal to that of the catalyst 57 in the third section 53, or lower than that of the catalyst 57 in the third section 53. The thickness of the first section 51 in the source gas flow direction is smaller than that of the third section 53 in the source gas flow direction. Thus, even when the catalytic activity of the catalyst 54 in the first section 51 is equivalent to the catalytic activity of the catalyst 57 in the third section 53, the temperature of the first section 51 attributed to heat of reaction can be lowered, as compared with the case in which the thickness of the first section 51 is greater than that of the third section 53. As a result, deterioration of the catalyst 54 can be suppressed.

By means of the condenser 56 disposed around the pipe 55 in the second section 52, the reaction product in the first section 51 is cooled to ice temperature, to thereby isolate water. Thus, since steam supplied to the third section 53 via the second section 52 can be reduced, the rate of reaction in the third section 53 can increase.

The present invention has been described on the basis of some embodiments. However, those skilled in the art can readily conceive that the present invention is not limited in any way to the aforementioned embodiments, and that various modifications and variations can be employed without deviating the scope of the present invention.

In the above embodiments, the cases in which the first section 21, 31, or 41, the second section 22, 32, or 42, and the third section 23, 33, or 43 have the same inner diameter were described. However, the inner diameter is not particularly limited thereto. Needless to say, the inner diameters of the first section 21, 31, or 41, the second section 22, 32, or 42, or the third section 23, 33, or 43 may be varied.

In the fourth embodiment, the case in which no heater is disposed around the condenser 56 provided in the second section 52 was described. However, the configuration is not particularly limited thereto. When the condenser 56 has such high cooling capacity as to suppress a rise in temperature of the second section 52 caused by a heater, the heater may be disposed around the condenser 56.

In the fourth embodiment, the case in which the second section 52 has the condenser 56 was described. However, the configuration is not particularly limited thereto. Needless to say, the condenser 56 may be omitted, since the rate of reaction in the second section 52 can be reduced as compared with the first section 51 or the third section 53 by connecting the first section 51 to the third section 53 via the pipe 55.

In the fourth embodiment, the case in which the condenser 56 is disposed around the pipe 55 provided in the second section 52 was described. However, the configuration is not particularly limited thereto. Needless to say, instead of the condenser 56, a low-activity catalyst having a catalytic activity lower than that of the catalyst 54 or 57, or an inert body having no catalytic activity may be disposed around the pipe 55.

### REFERENCE SIGNS LIST

10, 30, 40, 50 reaction device
20 reaction vessel
21, 31, 41, 51 first section
22, 32, 42, 52 second section
23, 33, 43, 53 third section
24, 26, 34, 36, 44, 46, 54, 57 catalyst
25, 45 low-activity catalyst
35 inert body

## Claims

1. A reaction device in which an exothermic chemical reaction occurs, the reaction device having a reaction vessel through which a source gas passes from an inlet to an outlet, and a catalyst accommodated in the reaction vessel,
wherein the reaction device includes a first section, a second section, and a third section disposed in that sequence along the direction of flow of the source gas; and
among the first, second, and third sections, the degree of lowering the activation energy of the chemical reaction is the smallest in the second section.

2. A reaction device in which an exothermic chemical reaction occurs, the reaction device having a reaction vessel through which a source gas passes from an inlet to an outlet, and a catalyst accommodated in the reaction vessel,
wherein the reaction device includes a first section, a second section, and a third section disposed in that sequence along the direction of flow of the source gas; and
the temperature rises in the first section, lowers in the second section, and rises in the third section.

3. A reaction device according to claim 1,
wherein the second section accommodates an inert body having no catalytic activity or a low-activity catalyst having a catalytic activity lower than that of a catalyst accommodated in the first section or the third section.

4. A reaction device according to claim 1 or 3,
wherein
the first section accommodates a catalyst having a catalytic activity lower than that of a catalyst accommodated in the third section.

5. A reaction device according to claim 1 or 3,
wherein the first section has a thickness in the gas flow direction smaller than the thickness of the third section in the gas flow direction.

6. A reaction device according to claim 1 or 2,
wherein the chemical reaction is a reaction of synthesizing methane from the source gas including hydrogen and carbon dioxide.
